(19) Europäisches Patentamt
European Patent Office
Office européen des brevets

(11) Publication number: 0 137 718
A2

(12) EUROPEAN PATENT APPLICATION

(21) Application number: 84306065.8

(22) Date of filing: 05.09.84

(51) Int. Cl.[4]: C 07 D 249/08
A 01 N 43/653

(30) Priority: 19.03.84 US 590880
06.09.83 US 529626

(43) Date of publication of application:
17.04.85 Bulletin 85/16

(84) Designated Contracting States:
AT BE CH DE FR GB IT LI LU NL SE

(71) Applicant: E.I. DU PONT DE NEMOURS AND COMPANY
1007 Market Street
Wilmington Delaware 19898(US)

(72) Inventor: Basarab, Gregory Steven
705 West 28th Street
Wilmington Delaware 19802(US)

(74) Representative: Hildyard, Edward Martin et al,
Frank B. Dehn & Co. Imperial House 15-19 Kingsway
London WC2B 6UZ(GB)

(54) **Fungicidal triazoleethanols.**

(57) Compounds of formula I

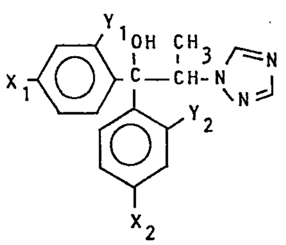

I

wherein

$X_1$, $X_2$, $Y_1$ and $Y_2$ are independently H, F. or Cl, with the proviso that at least one of $X_1$, $X_2$, $Y_1$ and $Y_2$ is F or Cl;

or fungicidally active salts of compounds of Formula I with protic acids or complexes of compounds of Formula I with metal ions;

exhibit potent fungical activity.

The novel compounds may be used to control fungal disease in plants by foliar application, soil application, or as a seed dressing.

The novel compounds may be made by reacting an appropriate 2-halo-1,1-diaryl-1-propanol or 1,1-diaryl-1-propylene oxide with 1,2,4-triazole.

EP 0 137 718 A2

"FUNGICIDAL TRIAZOLEETHANOLS"

This invention relates to novel fungicidal triazole-ethanols and their use for controlling fungus disease of plants.

US-A-4,414,210 very broadly discloses fungicidal compounds of the formula:

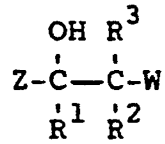

wherein Z is an aryl or substituted aryl group; $R^1$, $R^2$, and $R^3$, are independently H, cyano, alkyl, cycloalkyl, alkenyl, cycloalkenyl, alkynyl, aralkyl or substituted aralkyl, or aryl or substituted aryl, and W is 1 or 4-(1,2,4-triazole). In the above definitions, the "aryl" substituent is a $C_6$-$C_{10}$ aromatic, preferably phenyl or naphthyl, optionally substituted with up to three substituents selected from halogen, nitro, trihalomethyl, cyano, $C_1$-$C_4$ alkyl or alkylthio, $C_1$-$C_4$ alkylsulfinyl, $C_1$-$C_4$ alkylsulfonyl, phenoxy, phenylthio, phenylsulfinyl, phenylsulfonyl, or either of these last four substituted with up to two substituents selected from halogen, nitro, trifluoromethyl, cyano, methyl, methoxy, methylthio, methylsulfinyl, and methylsulfonyl.

GB-A-1,529,818 broadly discloses fungicidal compounds of the formula

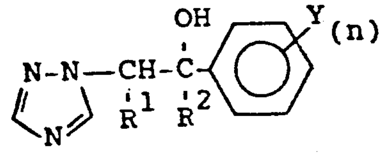

where $R^1$ and $R^2$ are independently hydrogen or optionally-substituted hydrocarbyl; Y is halogen, nitro, $C_1$-$C_4$ alkyl, or optionally substituted amino, and n is 1-5 (multiple Y being the same or different).

## Summary of the Invention

The present invention is directed to compounds of Formula I, agriculturally useful compositions of the compounds, and the method of using these compounds to control plant fungus diseases.

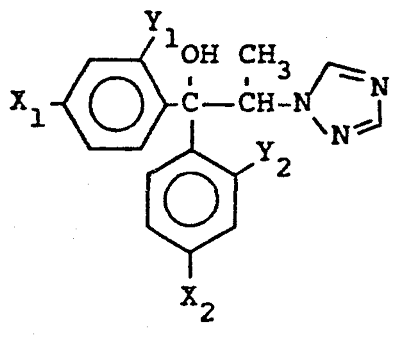

I

wherein

$X_1$, $X_2$, $Y_1$ and $Y_2$ are independently H, F, or Cl, with the proviso that at least one of $X_1$, $X_2$, $Y_1$ and $Y_2$ is F or Cl.

The present invention further relates to the fungicidally active salts of compounds of Formula I with protic acids and complexes of Formula I with metal ions.

Preferred for reasons of high fungicidal activity are compounds wherein at least one of $X_1$ and $Y_1$ and at least one of $X_2$ and $Y_2$ are independently Cl or F. More preferred are compounds wherein at least one of $X_1$ and $Y_1$ and at least one of $X_2$ and $Y_2$ are F. Specifically preferred is the compound α,α-bis(4-fluorophenyl)-ß-methyl-1H-1,2,4-triazole-1-ethanol.

The compounds of the present invention are also useful as intermediates for the production of the fungicidal compounds taught in our EP-A- of even date herewith, claiming priority from our U.S. Application Serial No. 529,626, filed 6 September 1983.

## Detailed Description of the Invention

### Synthesis

The Formula I compounds of this invention can be prepared by reacting either compounds of Formula II or Formula III with triazole potassium salt:

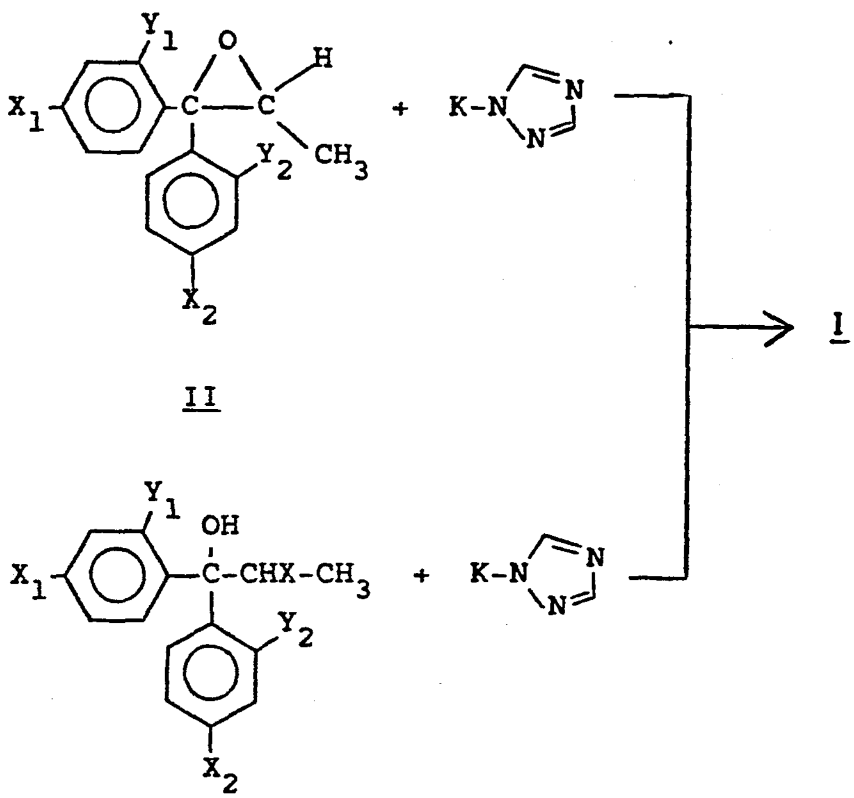

II

I

III

wherein X = Cl, Br or I and $X_1$, $X_2$, $Y_1$, and $Y_2$ are as defined above.

Lithium and sodium triazole salts may also be used. Approximately equimolar amounts of the reagents are used with the triazole salt often taken in excess of theory. In addition, triazole itself can be used if an acid acceptor is added. Suitable acceptors include alkali metal alkoxides such as sodium methoxide or potassium <u>tert</u>-butoxide, inorganic bases such as

potassium carbonate or sodium hydride, tertiary amines such as triethylamine and excess triazole. When the acid acceptor is a good nucleophile, such as sodium methoxide, an excess should be avoided to prevent undesired side reactions. Suitable solvents include protic solvents such as ethanol or ethylene glycol; polar, aprotic solvents such as dimethylformamide, dimethyl sulfoxide or acetonitrile; ethers such as tetrahydrofuran or 1,2-dimethoxyethane; and ketones such as 2-butanone. Addition of a suitable phase transfer catalyst such as a crown ether may enhance the reaction rate. Non-polar solvents, for example aromatic hydrocarbons such as toluene, may also be used if a suitable phase transfer catalyst such as tetrabutylammonium bromide is added. The reaction temperature can vary between 0° and 200°, preferably between 25° and 100°. The reactions can be conducted under elevated pressure, but it is generally preferable to operate at atmospheric pressure. The optimum temperature and reaction time will vary with the choice of solvent.

The intermediate compounds of general Formula II can be prepared by oxidation of compounds of Formula IV using methods set out in the literature:

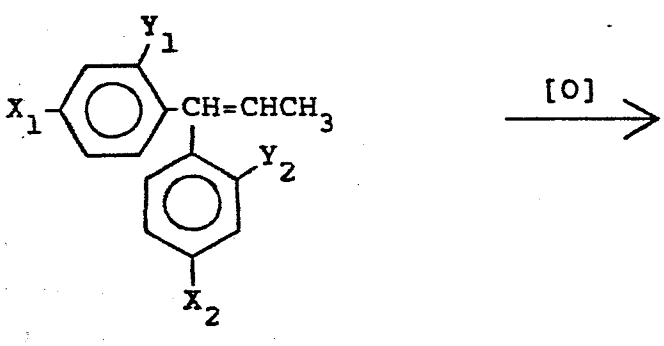


II

IV

wherein $X_1$, $Y_1$, $X_2$ and $Y_2$ are as defined above.

Suitable reagents which can effect this oxidation include peracids such as peracetic acid, m-chloroperbenzoic acid or p-nitroperbenzoic acid; hydroperoxides such as tert-butylhydroperoxide in the presence of an appropriate metal catalyst such as molybdenum hexacarbonyl; or hydrogen peroxide in combination with a nitrile such as acetonitrile or with an isocyanate such as phenyl isocyanate. Alternatively, the transformation of Formula IV compounds into Formula II compounds can be effected by first forming the halohydrin with hypochlorous acid or hypobromous acid and then reacting the intermediate halohydrin with a proton acceptor such as potassium tert-butoxide, sodium hydroxide or sodium hydride.

The intermediate compounds of general Formula IV may be prepared from compounds of general Formula V ($X_1$, $X_2$, $Y_1$ and $Y_2$ are as defined previously) by standard literature dehydration conditions. For example, reacting the appropriate Formula V compound with thionyl chloride in pyridine as solvent effects conversion to Formula IV compounds.

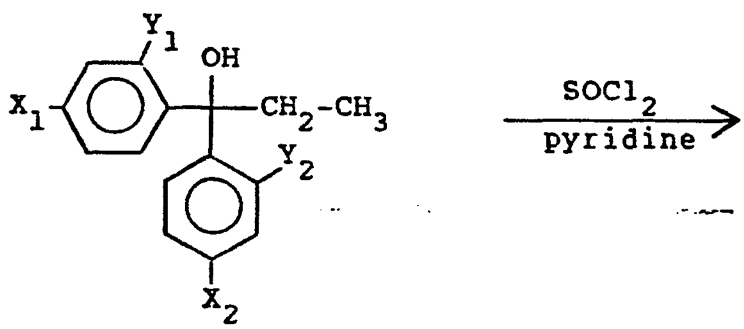


V

IV

The intermediate Formula IV compounds may also be prepared by reaction of benzophenone compounds of Formula VI, wherein $X_1$, $Y_1$, $X_2$ and $Y_2$ are as defined above, with triphenylphosphine ethylide.

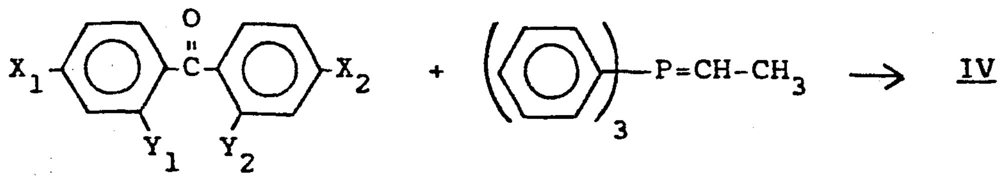

VI

Suitable solvents for this reaction include ether solvents such as tetrahydrofuran or ethyl ether. The triphenylphosphine ethylide is generated from an ethyltriphenylphosphonium halide (bromide, chloride or iodide) and a strong base such as butyllithium, potassium hydride or potassium _tert_-butoxide. The temperature of the reaction may range from -100° to 30°C depending on the solvent and on the strong base.

Compounds of Formula V can be prepared, in turn, by reacting the appropriate benzophenone compound of Formula VI, wherein $X_1$, $Y_1$, $X_2$ and $Y_2$ are as defined previously, with an ethylmagnesium halide, wherein Z is a halogen atom, preferably Cl, Br, or I, or with ethyllithium.

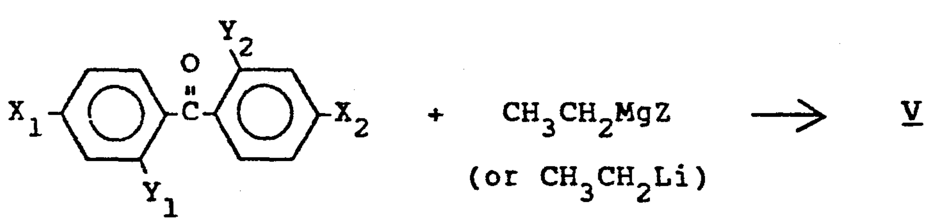


VI

This reaction is conducted in an ether solvent such as diethyl ether or tetrahydrofuran at a temperature of between 0°-60°C for the Grignard reagent and -70°-30°C for the lithium reagent.

In a second method for the preparation of Formula V compounds, the appropriate propiophenone compound of general Formula VII can be reacted with

the appropriate Grignard reagent (M=MgZ) or lithium reagent (M=Li) VIII wherein $X_1$, $X_2$, $Y_1$, $Y_2$ and Z are defined as previously described.

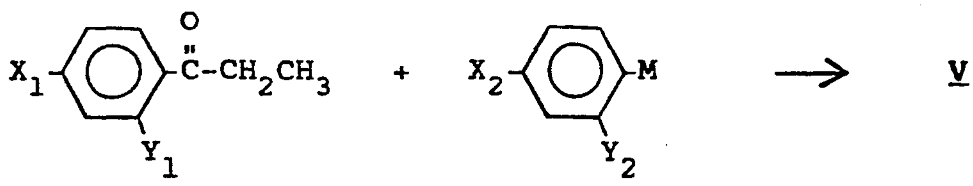

VII VIII V

The Grignard reactions are run in an ether solvent such as diethyl ether or tetrahydrofuran at temperatures of between 0°-60°C, depending on the identity of the Grignard reagent and the solvent. The lithium reactions are also run in an ether solvent at temperatures of between -100° to 0°C.

Intermediate compounds of general Formula III can be prepared by reacting a Grignard reagent VIII with a compound of general Formula IX wherein $X_1$, $X_2$, $Y_1$, $Y_2$, X and Z are as defined above:

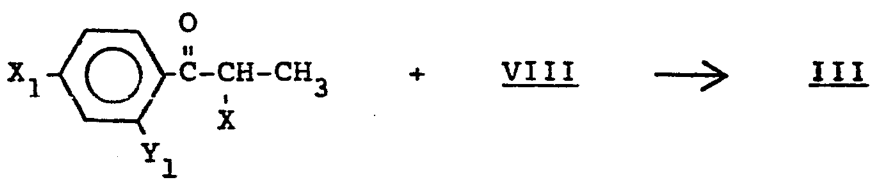

IX + VIII III

This reaction is run in an ether solvent such as diethyl ether or tetrahydrofuran at a temperature of between 0°-60°C, depending on the identity of the Grignard reagent and the solvent.

Those skilled in the art will recognize that the preparation of the triazole compounds of this invention can produce a mixture of two triazole isomers, the 1H-1,2,4-triazol-1-yl compound and the 4H-1,2,4-triazol-4-yl compound. Since the former isomer will be the predominant one, Formula I is drawn to depict its structure, and the compounds will be referred to as the 1H,1yl triazoles. It is to be recognized, however, that the 4H,4yl isomer can be present, and since this isomer is also fungicidally active, no separation of the isomers is necessary after preparation of the compounds.

It will be further recognized that the Formula I compounds can contain one or two asymmetric carbon atoms. The stereoisomers that can result all have fungicidal activity, and separation of these isomers is not required.

In the following examples, abbreviations for nuclear magnetic resonance (NMR) spectra are as follows: s = singlet; d = doublet, t = triplet; q = quartet; and m = multiplet. Peak positions for NMR are reported as parts per million downfield from the internal tetramethylsilane standard. Infrared (IR) peak positions are reported in reciprocal centimeters ($cm^{-1}$). Also, unless otherwise stated, ether refers to diethyl ether.

<u>Example 1a</u>

<u>Preparation of 1,1-bis(4-Fluorophenyl)-1-propanol</u>

A suspension of 4.0 g (0.164 mol) of magnesium turnings in 75 mL of tetrahydrofuran was stirred under nitrogen while a solution of 18 mL (0.164 mol) of 4-bromofluorobenzene in 75 mL of tetrahydrofuran was added dropwise with cooling in ice-water to maintain a temperature below 30°C. After stirring for 1.5 hours at room temperature, the solution was chilled in ice and a solution of 21.0 mL (0.151 mol) of <u>p</u>-fluoropropiophenone in 100 mL of dry tetrahydrofuran was added dropwise. The resulting mixture was stirred at room temperature for 16 hours, chilled in ice and

quenched with saturated aqueous ammonium chloride. After partitioning the reaction mixture between ether and water, the organic layer was separated, washed with brine and dried ($MgSO_4$). Removal of solvent afford the title compound as a viscous oil: $^1H$ NMR ($CDCl_3$): δ 0.86 (t, J=7Hz, 3H); 2.1 (s, 1H); 2.24 (q, J=7Hz, 2H), 7.0 (t, J=9Hz, 4H); 7.3 (d of d, J=9,6Hz, 4H).

## Example 1b

### Preparation of 1-(2-Fluorophenyl)-1-(4-fluorophenyl)-1-propanol

A suspension of 4.0 g (.164 mol) of magnesium turnings in 50 mL of ether was stirred under nitrogen while a solution of 13 mL (.174 mol) of ethylbromide was added dropwise with cooling in ice-water to maintain a temperature below 30°C. After stirring at room temperature for 1.5 hours, the solution was cooled in ice and a solution of 24.0 mL (0.137 mol) of 2,4'-difluorobenzophenone in 100 mL ether was added dropwise. The mixture was stirred at room temperature for 2.5 hours, chilled in ice, and quenched with saturated aqueous ammonium chloride. The ether layer was separated, washed with water and brine, dried ($MgSO_4$) and concentrated to a viscous yellow oil. Chromatography (4:1 hexanes-methylene chloride) on silica gel to elute the less polar of two components afforded the title compound as a viscous oil: $^1H$ NMR ($CDCl_3$): δ 0.9 (t, J=7Hz, 3H); 2.2 (m, 1H); 2.4 (m, 1H); 2.6 (s, 1H); 7.9-7.6 (m, 8H).

Following the procedures described earlier and exemplified by Examples 1a and 1b, the compounds in Table I can be prepared.

Table I

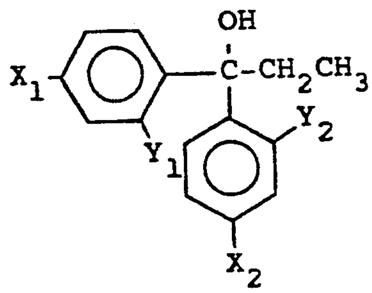

| $X_1$ | $Y_1$ | $X_2$ | $Y_2$ | Properties |
|---|---|---|---|---|
| Cl | Cl | H | H | |
| Cl | H | F | H | |
| H | Cl | H | Cl | |
| Cl | H | H | Cl | |
| F | F | F | H | |
| Cl | Cl | Cl | Cl | |
| H | Cl | H | H | |
| F | F | H | H | |
| F | H | H | H | |
| Cl | H | H | F | |
| F | H | H | Cl | |
| Cl | H | Cl | H | m.p. 38-43°C |

Example 2a

Preparation of 1,1-bis(4-Fluorophenyl)-1-propene

A solution of 34.0 g (.137 mol) of 1,1-bis(4-fluorophenyl)-1-propanol in 100 mL of pyridine was cooled to 0°C under nitrogen. Thionyl chloride (12.0 mL, 0.164 mol) was added dropwise over twenty minutes. The resulting mixture was stirred at room temperature for 2.5 hours, poured into 300 mL of ice chilled 2N hydrochloric acid and acidified by addition of concentrated hydrochloric acid. The mixture was extracted with three 100 mL portions of ether. The ether was dried ($MgSO_4$) and evaporated to afford the title compound which solidified upon distillation: bp 78-82°C (0.4 mm); m.p. 39-42°C; $^1H$ NMR ($CDCl_3$): $\delta$ 1.7 (d, J=7Hz, 3H); 6.1 (q, J=7Hz, 1H); 6.9 (t, J=9Hz, 2H); 7.1 (m, 6H).

Example 2b

Preparation of 1,1-bis(4-Fluorophenyl)-1-propene

A solution of ethyltriphenylphosphonium bromide (40.8 g, 11.0 mmol) in 200 mL of dry dimethylsulfoxide was cooled in an ice-bath while potassium tert-butoxide (12.3 g, 0.11 mol) was added portionwise. The solution was warmed to 20°C and 4,4'-difluorobenzophenone (21.8 g, 10.0 mmol) was added portionwise. The mixture was stirred at room temperature for 3 1/2 hours and 200 mL of water was added. The white solid which formed was filtered and the filtrate was extracted with two 200 mL portions of hexanes. The hexanes were washed with water and dried ($MgSO_4$). Removal of solvent gave the title compound which solidified upon distillation: m.p. 43-45°C.

Following the procedures described earlier and exemplified by Example 2, the compounds in Table II can be prepared.

Table II

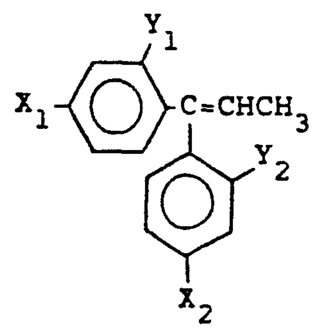

| $X_1$ | $Y_1$ | $X_2$ | $Y_2$ | Properties |
|---|---|---|---|---|
| Cl | Cl | H | H | |
| Cl | H | F | H | |
| H | Cl | H | Cl | |
| F | H | H | F | |
| Cl | H | H | Cl | |
| F | F | F | H | |
| Cl | Cl | Cl | Cl | |
| H | Cl | H | H | |
| F | F | H | H | |
| F | H | H | H | |
| Cl | H | H | F | |
| F | H | H | Cl | $n_D^{20.8}$ 1.5846 |
| Cl | H | Cl | H | $n_D^{20.8}$ 1.6062 |

Example 3a

Preparation of 1,1-bis(4-Fluorophenyl)-1-propylene oxide

To a suspension of 27.27 g (0.118 mol) of 1,1-bis(4-fluorophenyl)-1-propene and 25 g of solid sodium bicarbonate in 500 mL of methylene chloride was added 24.0 g (0.118 mol) of 85% m-chloroperbenzoic acid. The solution was stirred at room temperature 24 hours and washed with saturated aqueous sodium bisulfite, saturated aqueous sodium bicarbonate, water and brine. Drying ($MgSO_4$) and removal of solvent afforded the title compound as a viscous oil which crystallized: m.p. 75-80°C; $^1H$ NMR ($CDCl_3$) 1.2 (d, J=7Hz, 3H); 3.4 (q, J=7Hz, 1H); 6.9-7.8 (m, 8H).

Following the procedures described earlier and exemplified by Example 3a, the compounds in Table IIIa can be prepared.

Table IIIa

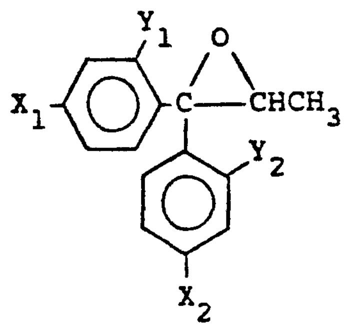

| $X_1$ | $Y_1$ | $X_2$ | $Y_2$ | Properties |
|---|---|---|---|---|
| Cl | Cl | H | H | |
| Cl | H | F | H | |
| H | Cl | H | Cl | |
| F | H | H | F | $n_D^{30.1}$ 1.5465 |
| Cl | H | H | Cl | |
| F | F | F | H | |
| Cl | Cl | Cl | Cl | |
| H | Cl | H | H | |
| F | F | H | H | |
| F | H | H | H | |
| Cl | H | H | F | |
| F | H | H | Cl | |
| Cl | H | Cl | H | m.p. 136-139°C |

Example 3b

Preparation of 2-Chloro-1,1-bis(4-chlorophenyl)-1-propanol

A suspension of 4.0 g (0.164 mol) of magnesium turnings in 75 mL of tetrahydrofuran is stirred under nitrogen while a solution of 31.4 gm (0.164 mol) of 4-bromochlorobenzene in 175 mL of tetrahydrofuran is added dropwise with cooling to maintain a temperature below 30°C. After stirring for 1.5 hours at room temperature, the solution is chilled in ice and a solution of 30.7 g (.151 mol) of 2,4-dichloropropiophenone in 100 mL of tetrahydrofuran is added dropwise. The resulting mixture is stirred at room temperature for 16 hours, chilled in ice and quenched with saturated aqueous ammonium chloride. After partitioning the reaction mixture between ether and water, the organic layer is separated, washed with brine and dried ($MgSO_4$). Removal of solvent affords the title compound.

Following the procedures described earlier and exemplified by Example 3b, the compounds in Table IIIb can be prepared.

Table IIIb

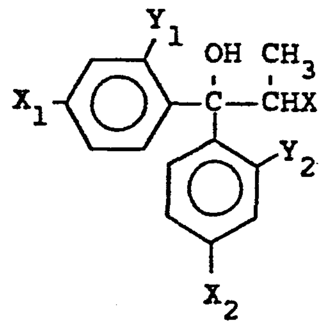

| $X_1$ | $Y_1$ | $X_2$ | $Y_2$ | X |
|---|---|---|---|---|
| F | F | H | H | Cl |
| Cl | H | F | H | Cl |
| H | Cl | H | Cl | Cl |
| F | H | H | F | Cl |
| Cl | H | H | Cl | Cl |
| F | F | F | H | Cl |
| F | H | F | H | Br |
| Cl | H | Cl | H | Br |
| F | H | F | H | I |
| Cl | H | Cl | H | I |

Example 4a

Preparation of α,α-bis(4-Fluorophenyl)-β-methyl-1H-1,2,4-triazole-1-ethanol

A solution of 1,1-bis(4-fluorophenyl)-1-propylene oxide (18.65 g, 76.0 mmol), potassium triazole (13.0 g, 121 mmol) and 18-crown-6 (1.5 g) in 200 mL of dimethylformamide was heated at 80-90°C under nitrogen for 20 hours. The solution was diluted with 350 mL of ether and washed three times with water and once with brine. Drying ($MgSO_4$) and removal of solvent gave a viscous yellow oil which solidified on standing. Trituration with hexanes afforded 11.7 g (49%) of title compound as a white solid: m.p. 147-149°C; $^1H$ NMR ($CDCl_3$): δ 1.6 (d, J=7Hz, 3H); 5.5 (s, 1H); 5.6 (q, J=7Hz, 1H); 6.8-7.8 (m, 8H); 7.8 (s, 1H); 8.0 (s, 1H).

Example 4b

Preparation of α,α-bis(4-Chlorophenyl)-β-methyl-1H-1,2,4-triazole-1-ethanol

A solution of 10.0 g (0.032 mol) of 2-chloro-1,1-bis(4-chlorophenyl)-1-propanol and 6.8 g (0.064 mol) of potassium triazole in 100 mL of dimethylformamide is heated at 80-90°C under nitrogen for 3 days. The solution is poured into water and extracted with ether. The ether is washed three times with water and once with brine. Drying ($MgSO_4$) and removal of solvent gives the title product which is purified by chromatography on silica gel: m.p. 111-112°C.

Following the procedures described earlier and exemplified by Examples 4a and 4b, the compounds in Table IV can be prepared.

Table IV

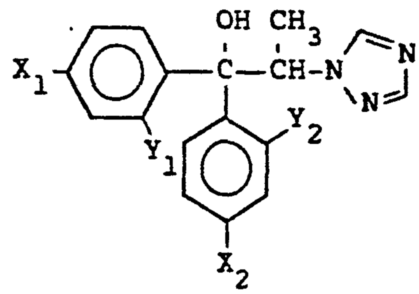

| $X_1$ | $Y_1$ | $X_2$ | $Y_2$ | m.p.(°C) |
|---|---|---|---|---|
| Cl | Cl | H | H | |
| Cl | H | F | H | 117-121 |
| H | Cl | H | Cl | |
| F | H | H | F | 110-112 |
| Cl | H | H | Cl | |
| F | F | F | H | |
| Cl | Cl | Cl | Cl | |
| H | Cl | H | H | |
| F | F | H | H | |
| F | H | H | H | |
| Cl | F | F | F | |
| F | H | H | Cl | |

Example 5

Preparation of the 1:1 Complex of α,α-bis(4-Chlorophenyl)-ß-methyl-1H-1,2,4-triazole-1-ethanol and cuprous chloride

A mixture of 3.5 g (0.01 mol) of α,α-bis(4-chlorophenyl)-ß-methyl-1H-1,2,4-triazole-1-ethanol and 1.0 g (0.01 mol) of cuprous chloride in 150 mL of tetrahydrofuran is refluxed under $N_2$ for 30 minutes, and the resulting deep green solution is evaporated to yield the title compound.

The following additional metal complexes can be prepared similarly using the appropriate metal salt:

1:1 complex with cupric chloride;
2:1 complex with cupric chloride;
1:1 complex with zinc chloride; and
1:1 complex with manganous sulfate.

Example 6

Preparation of the 4-Dodecylbenzenesulfonate Salt of α,α-bis(4-Chlorophenyl)-ß-methyl-1H-1,2,4-triazole-1-ethanol

A solution of 3.5 gm (0.01 mol) of α,α-bis(4-chlorophenyl)-ß-methyl-1H-1,2,4-triazole-1-ethanol in 30 mL of dichloromethane is combined with a solution of 3.3 g (0.01 mol) of 4-dodecylbenzenesulfonic acid in 30 mL of dichloromethane. The resulting solution is evaporated to yield the title compound.

The following additional acid complexes can be prepared similarly using the appropriate acid.

1:1 complex with hydrochloric acid;
1:1 complex with nitric acid; and
1:1 complex with trifluoroacetic acid.

Useful formulations of the compounds of Formula I can be prepared in conventional ways. They include dusts, granules, pellets, solutions, emulsions, wettable powders, emulsifiable concentrates and the like. Many of these may be applied directly. Sprayable formulations can be extended in suitable media and used at spray volumes of from as little as one up to several hundred liters or more per hectare. High strength compositions are primarily used as intermediates for further formulation. The formulations, broadly, contain about 1% to 99% by weight of active ingredient(s) and at least one of a) about 0.1% to 20% surfactant(s) and b) about 5% to 99% solid or liquid inert diluent(s). More specifically, they will contain these ingredients in the following approximate proportions:

| | Percent by Weight | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 5-50 | 40-95 | 0-15 |
| Aqueous Suspensions | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey. The more absorptive diluents are preferred for the wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", Allured Publ. Corp., Ridgewood, New Jersey, as well as Sisely and Wood, "Encyclopedia of Surface Active Agents", Chemical Publ. Co., Inc., New York, 1964, list surfactants and recommended uses. All formulations can contain minor amounts of additives to reduce foam, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, Dec. 4, 1967, pp. 147ff. and "Perry's Chemical Engineer's Handbook", 4th Edn., McGraw-Hill, N.Y., 1963, pp. 8-59ff.

For further information regarding the art of formulation, see for example:

H. M. Loux, U.S. Patent 3,235,361, Feb. 15, 1966, Col. 6, Line 16 through Col. 7, Line 19 and Examples 10 through 41.

R. W. Luckenbaugh, U.S. Patent 3,309,192, March 14, 1967, Col. 5, Line 43 through Col. 7, Line 62 and Examples 8, 12, 15, 39, 41, 52, 53, 58, 132, 138-140, 162-164, 166, 167, 169-182.

H. Gysin and E. Knusli, U.S. Patent 2,891,855, June 23, 1959, Col. 3, Line 66 through Col. 5, Line 17 and Examples 1-4.

G. C. Klingman, "Weed Control as a Science", John Wiley and Sons, Inc., New York, 1961, pp. 81-96.

J. D. Fryer and S. A. Evans, "Weed Control Handbook", 5th Edn. Blackwell Scientific Publications, Oxford, 1968, pp. 101-103.

J. B. Buchanan, U.S. Patent 3,576,834, April 27, 1971, Col. 5, Line 36, through Col. 7, Line 20 and Examples 1-4, 17, 106, 123-140.

R. R. Shaffer, U.S. Patent 3,560,616, February 2, 1971, Col, 3, Line 48, through Col. 7, Line 26 and Examples 3-9, 11-18.

E. Somers, "Formulation", Chapter 6 in Torgeson, "Fungicides", Vol. I, Academic Press, New York, 1967.

Examples of useful formulations of compounds of the present invention are as follows.

## Example 7

Wettable Powder

| | |
|---|---|
| α,α-bis(4-fluorophenyl)-ß-methyl-1H-1,2,4-triazole-1-ethanol | 80% |
| sodium alkylnaphthalenesulfonate | 2% |
| sodium ligninsulfonate | 2% |
| synthetic amorphous silica | 3% |
| kaolinite | 13% |

The ingredients are blended and hammer-milled.

Example 8

High Strength Concentrate

| | |
|---|---|
| α,α-bis(4-fluorophenyl)-β-methyl-1H-1,2,4-triazole-1-ethanol | 95% |
| silica aerogel | 0.5% |
| synthetic amorphous fine silica | 4.5% |

The ingredients are blended and ground in a hammer-mill to produce a high strength concentrate essentially all passing a U.S.S. No. 50 sieve (0.3 mm openings). This material can then be formulated in a variety of ways.

Example 9

Dust

| | |
|---|---|
| wettable powder of Example 7 | 10% |
| pyrophyllite (powder) | 90% |

The wettable powder and the pyrophyllite diluent are thoroughly blended and then packaged. The product is suitable for use as a dust.

Example 10

Aqueous Suspension

| | |
|---|---|
| α,α-bis(4-fluorophenyl)-β-methyl-1H-1,2,4-triazole-1-ethanol | 50% |
| dodecylphenol polyethylene glycol ether | 0.5% |
| crude calcium ligninsulfonate | 5.0% |
| xantham gum thickener | 0.2% |
| paraformaldehyde | 0.2% |
| water | 44.1% |

The ingredients are ground together in a sand, ball, or roller mill to produce particles essentially all under five microns in size.

Example 11

Solution

| | |
|---|---|
| α,α-bis(4-fluorophenyl)-β-methyl-1H-1,2,4-triazole-1-ethanol | 30% |
| dimethylformamide | 70% |

The ingredients are combined and stirred to produce a solution, which can be used for low volume applications.

Example 12

Emulsifiable Concentrate

| | |
|---|---|
| α,α-bis(4-fluorophenyl)-β-methyl-1H-1,2,4-triazole-1-ethanol | 30% |
| blend of oil soluble sulfonates and polyoxyethylene ethers | 4% |
| xylene | 66% |

The ingredients are combined and stirred until the active is dissolved. A fine screen filter is included in packaging operation to insure the absence of any extraneous undissolved material in the product.

Example 13

Granule

| | |
|---|---|
| wettable powder of Example 7 | 80% |
| sugar | 20% |

The ingredients are blended in a rotating or fluid bed mixer and water sprayed on to accomplish granulation. When most of the material has reached the desired range of 1.0 to 0.42 mm. (U.S.S. No. 18 to 40 sieves), the granules are removed, dried, and screened. Oversize material is crushed to produce additional material in the desired range. These granules contain 64% active ingredient.

Example 14

Dust Seed Coat

| | |
|---|---|
| α,α-bis(4-fluorophenyl)-β-methyl-1H-1,2,4-triazole-1-ethanol | 75% |
| permanent red 2 B, calcium salt, extended on Blanc Fixe | 5% |
| diatomaceous earth | 20% |

The ingredients are blended, coarsely hammer milled and passed through a fluid mill to produce

particles of active ingredient that are essentially all below 10 microns in diameter. The product is reblended before packaging. (Product is used by dusting on seed at rates of 1-800 gms/100 kg.)

Example 15

Slurry Seed Coat

| | |
|---|---|
| α,α-bis(4-fluorophenyl)-β-methyl-1H-1,2,4-triazole-1-ethanol | 65% |
| calcium ligninsulfonate | 4% |
| trimethyl nonyl polyethylene glycol ether | 4% |
| Rhodamine B | 1% |
| permanent red 2 B, calcium salt, extended on Blanc Fixe | 1% |
| diatomaceous earth | 25% |

The liquid surfactant is sprayed on the diatomaceous earth, the other ingredients are then added and thoroughly mixed together in an efficient blender. The mixture is then coarsely hammer milled and passed through a fluid mill to produce particles of active ingredient that are essentially all less than 10 microns in diameter. The product is reblended before packaging. (Product is used by making about a 30-40% slurry in water and applying the slurry to seed in a commercial seed treater.)

Utility

The compounds of this invention are useful as plant disease control agents. They are effective in controlling a broad spectrum of plant diseases, particularly foliar pathogens of ornamental, vegetable, field, cereal and fruit crops, such as, *Puccinia recondita*, *Erysiphe graminis*, *Bipolaris oryzae*, *Venturia inaequalis*, *Sphaerotheca fuliginea*, *Cercosporidium personatum*, *Monilinia fructicola*, and soil-borne pathogens, such as *Rhizoctonia solani*, *Helminthosporium sativum*, and *Thielaviopsis basicola*.

Disease control is ordinarily accomplished by applying an effective amount of the compound, normally as part of a formulation containing it, either pre- or post-infection to the portion of the plant to be protected, such as the roots, stems, foliage, fruit, seeds, tubers or bulbs, or to the medium (soil or sand) in which the plants to be protected are growing. The compound may also be applied to the seed from which the plants to be protected are to be grown.

Rates of application for these compounds can be influenced by many factors of the environment and should be determined under actual use conditions. Foliage can normally be protected when treated at a rate of from less than 1 to 500 grams of active ingredient per hectare. Plants growing in soil treated at a concentration from 0.1 to about 20 kg/ha can be protected from disease. Seed and seedlings can normally be protected when seed is treated at a rate of from 0.06 to about 3 grams per kilogram of seed.

The compounds of this invention can be mixed with fungicides, bactericides, acaricides, nematicides, insecticides, or other biologically active compounds in order to achieve desired results with a minimum expenditure of time, effort and material. Amounts of these biologically active materials added for each part by weight of the compound of this invention may vary from 0.05 to 25 parts by weight. Suitable agents of this type are well-known to those skilled in the art. Some are listed below:

<u>Fungicides</u>:

methyl 2-benzimidazolecarbamate (carbendazim)
tetramethylthiuram disulfide (thiuram)
*n*-dodecylguanidine acetate (dodine)
manganese ethylenebisdithiocarbamate (maneb)
1,4-dichloro-2,5-dimethoxybenzene (chloroneb)

methyl 1-(butylcarbamoyl)-2-benzimidazolecarbamate (benomyl)

2-cyano-N-ethylcarbamoyl-2-methoxyiminoacetamide (cymoxanil)

N-trichloromethylthiotetrahydrophthalimide (captan)

N-trichloromethylthiophthalimide (folpet)

dimethyl(4,4'-o-phenylene)bis(3-thioallophanate) (thiophanate-methyl)

2-(thiazol-4-yl)benzimidazole (thiabendazole)

aluminum tris(O-ethyl phosphonate) ("Aliette")

tetrachloroisophthalonitrile (chlorothalonil)

2,6-dichloro-4-nitroaniline (dichloran)

N-(2,6-dimethylphenyl)-N-(methoxyacetyl)alanine methyl ester (metalaxyl)

cis-N-[(1,1,2,2-tetrachloroethyl)thio]-4-cyclohexene-1,2-dicarbioximide (captafol)

3-(3,5-dichlorophenyl)-N-(1-methyl-ethyl)-2,4-dioxo-1-imidazolidine carboxamide (iprodione)

3-(3,5-dichlorophenyl)-5-ethenyl-5-methyl-2,4-oxazolidinedione (vinclozolin)

kasugamycin

O-ethyl S,S-diphenyl phosphorodithioate (edifenphos)

Bactericides:

tribasic copper sulfate

streptomycin sulfate

oxytetracycline

Acaricides:

senecioic acid, ester with 2-sec-butyl-4,6-dinitrophenol (binapacryl)

6-methyl-1,3-dithiolo[2,3-ß]quinonolin-2-one (oxythioquinox)

2,2,2-trichloro-1,1-bis(4-chlorophenyl)ethanol (dicofol)

bis(pentachloro-2,4-cyclopentadien-1-yl) (dienochlor)

tricyclohexyltin hydroxide (cyhexatin)

hexakis(2-methyl-2-phenylpropyl)distannoxane (fenbutin oxide)

<u>Nematicides</u>:

2-[diethoxyphosphinylimino]-1,3-dithietane (fosthietan)

S-methyl-1-(dimethylcarbamoyl)-N-(methylcarbamoyloxy)-thioformimidate (oxamyl)

S-methyl-1-carbamoyl-N-(methylcarbamoyloxy)thioformimidate

N-isopropylphosphoramidic acid, O-ethyl-O'-[4-(methylthio)-*m*-tolyl]diester (fenamiphos)

<u>Insecticides</u>:

3-hydroxy-N-methylcrotonamide(dimethylphosphate)ester (monocrotophos)

methylcarbamic acid, ester with 2,3-dihydro-2,2-dimethyl-7-benzofuranol (carbofuran)

O-[2,4,5-trichloro-α-(chloromethyl)benzyl]phosphoric acid, O',O'-dimethyl ester (tetrachlorvinphos)

2-mercaptosuccinic acid, diethyl ester, S-ester with thionophosphoric acid, dimethyl ester (malathion)

phosphorothioic acid, O,O-dimethyl, O-*p*-nitrophenyl ester (methyl parathion)

methylcarbamic acid, ester with α-naphthol (carbaryl)

methyl N-[[(methylamino)carbonyl]oxy]ethanimidothioate (methomyl)

N'-(4-chloro-*o*-tolyl)-N,N-dimethylformamidine (chlordimeform)

O,O-diethyl-O-(2-isopropyl-4-methyl-6-pyrimidyl)phosphorothioate (diazinon)

octachlorocamphene (toxaphene)

O-ethyl O-*p*-nitrophenyl phenylphosphonothioate (EPN)

cyano(3-phenoxyphenyl)-methyl 4-chloro-α-(1-methylethyl)benzeneacetate (fenvalerate)

(3-phenoxyphenyl)methyl (±)-*cis,trans*-3-(2,2-dichloroethenyl)-2,2-dimethylcyclopropanecarboxylate (permethrin)

dimethyl N,N'-[thiobis][N-methylimino)carbonyloxy]]-bis[ethanimidothioate] (thiodicarb)

phosphorothiolothionic acid, O-ethyl-O-[4-(methylthio)-phenyl]-S-n-propyl ester (sulprofos)

α-cyano-3-phenoxybenzyl 3-(2,2-dichlorovinyl)-2,2-dimethylcyclopropane carboxylate (cypermethrin)

cyano(3-phenoxyphenyl)methyl 4-(difluoromethoxy)-α-(methylethyl)benzeneacetate ("Payoff")

O,O-diethyl-O-(3,5,6-trichloro-2-pyridyl)phosphorothioate (chlorpyrifos)

O,O-dimethyl-S-[(4-oxo-1,2,3-benzotriazin-3-(4H)-yl)-methyl]phosphorodithioate (azinphos-methyl)

5,6-dimethyl-2-dimethylamino-4-pyrimidinyl dimethyl carbamate ("Pirimor")

S-(N-formyl-N-methylcarbamoylmethyl-O,O-dimethyl phosphorodithioate (formothion)

S-2-(ethylthioethyl)-O,O-dimethyl phosphiorothioate (demeton-S-methyl)

α-cyano-3-phenoxybenzyl cis-3-(2,2-dibromovinyl)-2,2-dimethylcyclopropane carboxylate (deltamethrin)

cyano(3-phenoxyphenyl)methyl ester of N-(2-chloro-4-trifluoromethylphenyl)alanine ("Mavrik")

This invention is further illustrated by the following examples.

## Example 16

A compound of this invention was dissolved in acetone in an amount equal to 6% of the final volume and then made into a spray formulation by suspension in purified water containing 250 ppm of the surfactant TREM 014 (polyhydric alcohol esters). This compound was sprayed at a concentration of 93 grams/hectare (935 liters of the formulation per hectare) on wheat seedlings. The following day, the plants were inoculated with a spore suspension of <u>Puccinia recondita</u>

var. tritici, causal agent of wheat leaf rust, and incubated in a saturated humidity chamber at 20° for 24 hours and then in a growth room for an additional 7 days, when disease ratings were made. Percent disease control is shown in the following table. Treated plants had no rust pustules while the untreated plants had numerous rust pustules on each leaf.

Table V

| Compound | % Control Wheat Rust |
|---|---|
| α,α-bis(4-fluorophenyl)-β-methyl-1H-1,2,4-triazole-1-ethanol | 100 |

Example 17

A compound of this invention was dissolved in acetone in an amount equal to 6% of the final volume and then made into a spray formulation by suspension in purified water containing 250 ppm of the surfactant TREM 014 (polyhydric alcohol esters). This compound was sprayed at a concentration of 93 grams/hectare (935 liters of the formulation per hectare) on wheat seedlings. The following day, the plants were inoculated with a spore suspension of Erysiphe graminis var. tritici, causal agent of wheat powdery mildew, and incubated in a growth room for 14 days, when disease ratings were made. Percent disease control is shown in the following table. Treated plants had no powdery mildew while the untreated plants were covered with powdery mildew.

Table VI

| Compound | % Control Wheat Powdery Mildew |
|---|---|
| α,α-bis(4-fluorophenyl)-β-methyl-1H-1,2,4-triazole-1-ethanol | 100 |

Example 18

A compound of this invention was dissolved in acetone in an amount equal to 6% of the final volume and then made into a spray formulation by suspension in purified water containing 250 ppm of the surfactant TREM 014 (polyhydric alcohol esters). This compound was sprayed at a concentration of 93 grams/hectare (935 liters of the formulation per hectare) on rice seedlings. The following day, the plants were inoculated with a spore suspension of _Bipolaris_ _oryzae_, causal agent of rice brown leaf spot, and incubated in a saturated humidity chamber at 20° for 24 hours and then in a growth room for an additional 7 days, when disease ratings were made. Percent disease control is shown in the following table. Treated plants had no lesions while the untreated plants had numerous lesions on each leaf.

Table VII

| Compound | % Control of Rice Brown Leaf Spot |
|---|---|
| α,α-bis(4-fluorophenyl)-β-methyl-1H-1,2,4-triazole-1-ethanol | 100 |

Example 19

A compound of this invention was dissolved in acetone in an amount equal to 6% of the final volume and then made into a spray formulation by suspension in purified water containing 250 ppm of the surfactant TREM 014 (polyhydric alcohol esters). This compound was sprayed at a concentration of 93 grams/hectare (935 liters of the formulation per hectare) on apple seedlings. The following day, the plants were inoculated with a spore suspension of the fungus *Venturia inaequalis*, causal agent of apple scab, and incubated in a saturated humidity chamber at 20° for 24 hours and then in a growth room for an additional 10-12 days. Disease ratings were then made and recorded as shown in the following table. Treated plants had no apple scab lesions when compared to untreated plants which were covered with scab lesions.

Table VIII

| Compound | % Control Apple Scab |
|---|---|
| α,α-bis(4-fluorophenyl)-ß-methyl-1H-1,2,4-triazole-1-ethanol | 100 |

Example 20

A compound of this invention was dissolved in acetone in an amount equal to 6% of the final volume and then made into a spray formulation by suspension in purified water containing 250 ppm of the surfactant TREM 014 (polyhydric alcohol esters). This compound was sprayed at a concentration of 93 grams/hectare (935 liters of the formulation per hectare) on cucumber seedlings. The following day, the plants were inoculated with a spore suspension of the fungus

Sphaerotheca fuliginea, causal agent of cucumber powdery mildew, and incubated in a growth room for 7 days. Disease ratings were then made. Percent disease control is shown in the following table. Treated plants had no powdery mildew in contrast to untreated plants which were covered with powdery mildew.

Table IX

| Compound | % Control Cucumber Powdery Mildew |
|---|---|
| α,α-bis(4-fluorophenyl)-β-methyl-1H-1,2,4-triazole-1-ethanol | 100 |

Example 21

A compound of this invention was dissolved in acetone in an amount equal to 6% of the final volume and then made into a spray formulation by suspension in purified water containing 250 ppm of the surfactant TREM 014 (polyhydric alcohol esters). This compound was sprayed at a concentration of 93 grams/hectare (935 liters of the formulation per hectare) on peanut seedlings. The following day, the plants were inoculated with a spore suspension of Cercosporidium personatum, causal agent of peanut late leafspot, and incubated in a saturated humidity chamber at 27° for 24 hours and then in a growth room for an additional 14 days, when disease ratings were made. Percent disease control is shown in the following table. Treated plants had no leafspots while the untreated plants had numerous leafspots.

Table X

| Compound | % Control of Peanut Late Leafspot |
|---|---|
| α,α-bis(4-fluorophenyl)-ß-methyl-1H-1,2,4-triazole-1-ethanol | 100 |

Example 22

A compound of this invention was dissolved in acetone so that 1 ml of solution yielded a concentration of 0.1 gram/kilogram of seed when evenly coated on 5 grams of wheat seed. Treated seeds were air dried, planted, incubated in a growth room for 14 days, then inoculated with a spore suspension of the fungus Puccinia recondita var. tritici, causal agent of wheat leaf rust. Inoculated plants were incubated in a saturated humidity chamber at 20° for 24 hours and then in a growth room for an additional 7 days, when disease ratings were made. Percent disease control is shown in the following table. Plants grown from treated seed had no rust pustules while plants from untreated seed had numerous rust pustules on each leaf.

Table XI

| Compound | % Control Wheat Rust |
|---|---|
| α,α-bis(4-fluorophenyl)-ß-methyl-1H-1,2,4-triazole-1-ethanol | 100 |

Example 23

A compound of this invention was dissolved in acetone so that 1 ml of solution yielded a concentration of 0.1 gram/kilogram of seed when evenly coated on 5 grams of wheat seed. Treated seeds were air

dried, planted, incubated in a growth room for 14 days, then inoculated with a spore suspension of the fungus Erysiphe graminis var. tritici, causal agent of wheat powdery mildew, and then further incubated in a growth room for an additional 7 days. Disease ratings were then made. Percent disease control is shown in the following table. Plants grown from treated seed had no powdery mildew in contrast to plants from untreated seed which were covered with powdery mildew.

Table XII

| Compound | % Control Wheat Powdery Mildew |
|---|---|
| α,α-bis(4-fluorophenyl)-β-methyl-1H-1,2,4-triazole-1-ethanol | 100 |

Example 24

A compound of this invention was dissolved in acetone in an amount equal to 5% of the final volume and then suspended at a concentration of 23 ppm in purified water containing 700 ppm of the surfactant TREM 014 (polyhydric alcohol esters). Canned peach halves were dipped in this suspension for three minutes and then placed to air dry in sterile containers. Upon drying, the peach halves were inoculated with two pieces of Monilinia fructicola mycelium, causal agent of stone fruit brown rot, and incubated in the sterile containers for five days. At that time the radii of the colonies' growth were measured on each peach. Colonies on treated peaches did not grow or grew only a few milliliters in diameter while those growing on untreated peaches covered the entire surface of the peach. Percent disease contol (percent growth inhibition of colonies

on treated peaches as compared to that of colonies on untreated peaches) is expressed in the table below.

Table XIII

| Compound | % Control Brown Rot |
|---|---|
| α,α-bis(4-fluorophenyl)-ß-methyl-1H-1,2,4-triazole-1-ethanol | 98 |

Example 25

A compound of this invention was dissolved in acetone in an amount equal to 6% of the final volume and then made into a spray formulation by suspension in purified water containing 250 ppm of the surfactant TREM 014 (polyhydric alcohol esters). This compound was sprayed at a concentration of 93 grams/hectare (935 liters of the formulation per hectare) on rice seedlings. The following day, the plants were inoculated with a mixture of bran and the mycelium of the fungus _Rhizoctonia solani_, causal agent of sheath blight of rice, and incubated in a growth room for 7 days. Disease ratings were then made. Percent disease control is shown in the following table. Treated plants had no sheath blight in contrast to untreated plants which were covered with sheath blight.

Table XIV

| Compound | % Control of Rice Sheath Blight |
|---|---|
| α,α-bis(4-fluorophenyl)-ß-methyl-1H-1,2,4-triazole-1-ethanol | 100 |

Example 26

A compound of this invention was dissolved in acetone so that 1 ml of solution yielded a concentration of 0.2 gram/kilogram of seed when evenly coated on 5 grams of wheat seed. Treated seeds in soil were then inoculated with a mixture of sand, cereal and mycelium of the fungus Helminthosporium sativum, causal agent of wheat root rot, and incubated in a growth room for 14 days. Disease ratings were made and percent disease control is shown in the following table. Plants grown from treated seed had no root rot in contrast to plants from untreated seed which were heavily rotted.

Table XV

| Compound | % Control of Wheat Root Rot |
|---|---|
| α,α-bis(4-fluorophenyl)-β-methyl-1H-1,2,4-triazole-1-ethanol | 100 |

Example 27

A compound of this invention was dissolved in acetone and applied to cotton seeds and soil in pots at a concentration equivalent to 5.0 kilogram/hectare. Seeds and soil were then inoculated with a mixture of sand, cereal and mycelium of the fungus Thielaviopsis basicola, causal agent of cotton black root rot, and incubated in a growth room for 14 days. Disease ratings were made and percent disease control is shown in the following table. Treated seeds germinated well and had little root rot in contrast to the untreated seeds which germinated but were rotted by 14 days.

Table XVI

| Compound | % Control of Cotton Black Root Rot |
|---|---|
| α,α-bis(4-fluorophenyl)-β-methyl-1H-1,2,4-triazole-1-ethanol | 96 |

Claims:

1. A compound of Formula I:

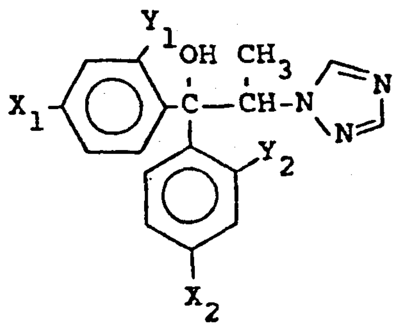

I

wherein

$X_1$, $X_2$, $Y_1$ and $Y_2$ are independently H, F, or Cl, with the proviso that at least one of $X_1$, $X_2$, $Y_1$ and $Y_2$ is F or Cl;

or fungicidally active salts of compounds of Formula I with protic acids or complexes of compounds of Formula I with metal ions.

2. A compound of Claim 1 wherein at least one of $X_1$ and $Y_1$ and at least one of $X_2$ and $Y_2$ are independently Cl or F.

3. A compound of Claim 2 wherein at least one of $X_1$ and $Y_1$ and at least one of $X_2$ and $Y_2$ are F.

4. The compound of Claim 1 that is α,α-bis(4-fluorophenyl)-β-methyl-1H-1,2,4-triazole-1-ethanol, or a fungicidally active salt of said compound with a protic acid or a complex of said compound with a metal ion.

5. A composition for controlling fungus disease in plants which comprises an effective amount of a compound of any of claims 1 to 3 and at least one of the following: surfactant: solid inert diluent; or liquid inert diluent.

6. A composition for controlling fungus disease in plants which comprises an effective amount of the compound of claim 4 and at least one of the following: surfactant; solid inert diluent; or liquid inert diluent.

7. A method for controlling fungus disease in plants which comprises applying to the locus to be protected an effective amount of a compound of any of claims 1 to 3.

8. A method for controlling fungus disease in plants which comprises applying to the locus to be protected an effective amount of the compound of claim 4.

9. Seed dressed with a fungicidally effective amount of a compound of any of claims 1 to 4.

10. A method for the preparation of a compound of claim 1 which comprises reacting a compound of formula

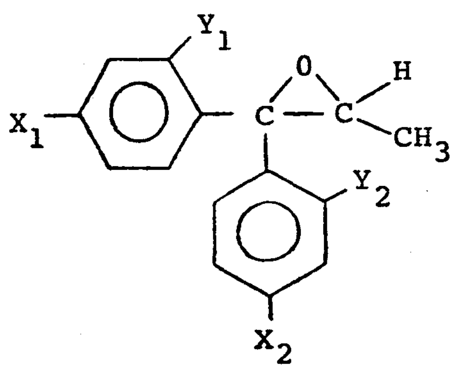

or

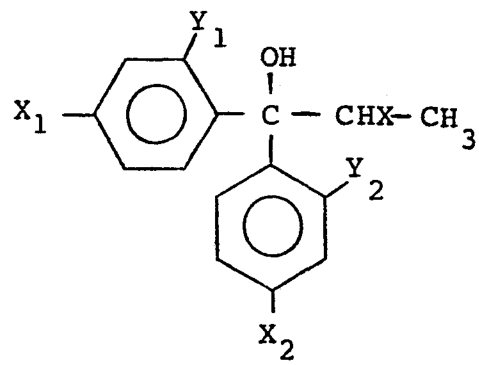

(II) (III)

wherein X is Cl, Br or I and $X_1$, $X_2$, Y and $Y_2$ are as defined in claim 1, with

(a) an alkali metal salt of 1,2,4-triazole; or

(b) 1,2,4-triazole in the presence of an acid acceptor;

whereafter if desired the product of formula (I) is converted into a fungicidally active salt thereof with a protic acid, or into a complex thereof with a metal ion.

For the designated contracting state AT



Claims:

1. A method for the preparation of a compound of Formula I:

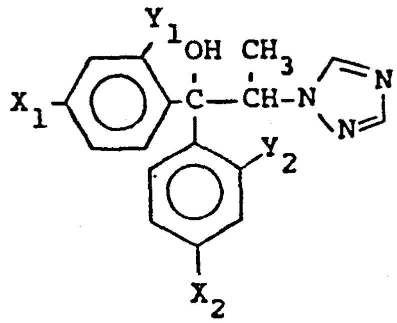

I

wherein

$X_1$, $X_2$, $Y_1$ and $Y_2$ are independently H, F, or Cl, with the proviso that at least one of $X_1$, $X_2$, $Y_1$ and $Y_2$ is F or Cl;

or a fungicidally active salt of said compound with a protic acid or a complex of said compound with a metal ion; which comprises reacting a compound of formula

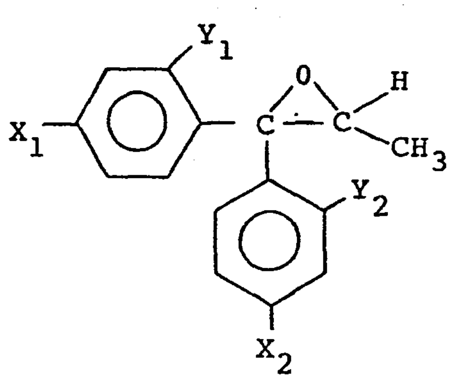

or

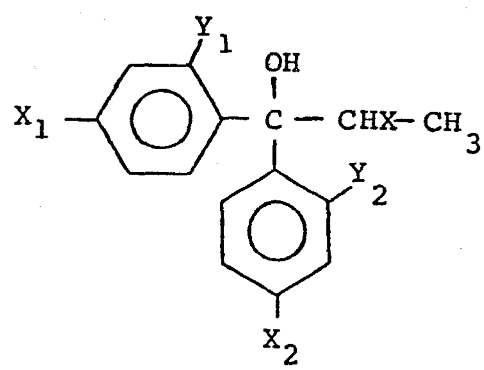

(II) (III)

wherein X is Cl, Br or I and $X_1$, $X_2$, Y and $Y_2$ are as defined above, with

(a) an alkali metal salt of 1,2,4-triazole; or

(b) 1,2,4-triazole in the presence of an acid acceptor;

whereafter if desired the product of formula (I) is converted into a fungicidally active salt thereof with a protic acid, or into a complex thereof with a metal ion.

2. A process of claim 1 wherein said reaction is performed in a solvent medium at a temperature between 25° and 100°C, optionally in the presence of a phase transfer catalyst.

3. A process of claim 1 or 2 wherein at least one of $X_1$ and $Y_1$ and at least one of $X_2$ and $Y_2$ are independently Cl or F.

4. A process of claim 3 wherein at least one of $X_1$ and $Y_1$ and at least one of $X_2$ and $Y_2$ are F.

5. A process of claim 1 wherein $X_1$ and $X_2$ are F and $Y_1$ and $Y_2$ are H.

6. A composition for controlling fungus disease in plants which comprises an effective amount of a compound of formula (I) as defined in any of claims 1, 3, 4, or 5 and at least one of the following: surfactant; solid inert diluent; or liquid inert diluent.

7. Seed dressed with a fungicidally effective amount of a compound of formula (I) as defined in any of claims 1, 3, 4 or 5.

8. A method of controlling fungus disease in plants which comprises applying to the locus to be protected an effective amount of a compound of formula (I) as defined in any of claims 1, 3, 4 or 5.

9. The method of claim 8 wherein said compound comprises α,α-bis(4-fluorophenyl)-β-methyl-1H-1,2,4-triazole-1-ethanol, or a fungicidally active salt thereof with a protic acid or a complex thereof with a metal ion.

10. The method of claim 8 or 9 wherein said compound is applied to the foliage of said plants, to the soil, or as a seed dressing.